# EUROPEAN PATENT APPLICATION

(11) **EP 2 159 228 A1**
(43) Date of publication of application: **03.03.2010**
(21) Application number: 08252848.0
(22) Date of filing: 28.08.2008
(51) Int. Cl.: C07K 1/04, C08G 81/00, C08G 81/02

(54) **Amphiphilic solid support**

(71) Applicant: Polymer Laboratories Limited, 115 Colmore Row Birmingham B3 3AL (GB)
(72) Inventor: Marsh, Peter A., Shrewsbury, Shropshire, SY3 9HX (GB); Davies, John W., Bayston Hill, Shrewsbury, Shropshire, SY3 ONZ (GB)
(74) Representative: Hartley, Andrew Philip

(57) **Abstract**

Amphiphilic solid supports for solid phase synthesis are disclosed, and methods for preparation and use thereof, comprising a solid support and a compound according to Formula I covalently bound thereto: 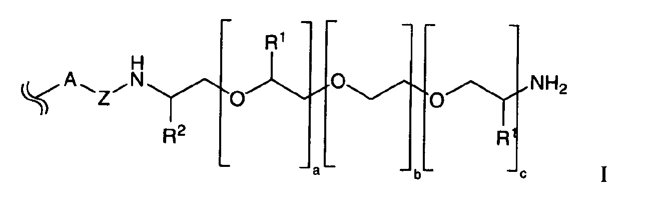 wherein A is C₅₋₁₀ aryl or heteroaryl, Z is either lower alkyl or absent, R¹ is lower alkyl, R² is H or lower alkyl, a is an integer from about 0 to about 20, b is an integer from about 5 to about 150, c is an integer from about 0 to about 20.

## Description

### FIELD OF THE INVENTION

This invention relates generally to compositions and methods for synthesizing polymers, such as polynucleotides, polypeptides, small molecules, and the like.

### BACKGROUND OF THE INVENTION

Substrates for use in solid phase synthesis of peptides have been the object of numerous experimental investigations in recent years. Conventional solid phase peptide synthesis (SPPS) used low crosslinked (1-2% divinylbenzene) chloromethyl polystyrene (Merrifield, R.B. (1962) Fed. Proc. Fed. Am. Soc. Exp. Biol. 21, 412). During the early years of SPPS using polystyrene supports, some peptide syntheses were identified collectively as difficult sequences to prepare. The problem occurring in these difficult sequences was postulated to be due to the hydrophobic environment of polystyrene lending itself toward aggregation of the growing peptide chains via intramolecular hydrogen-bonding, either via intramolecular coiling or intermolecular interactions with neighbouring peptide chains. These secondary structure effects limit the accessibility of coupling reagent to the growing peptide reactive site and lead to sequence dependent coupling deficiencies.

The suggested incompatibility between the non-polar polystyrene and the polar peptide chains led to the development of alternative supports where the peptide and support are more compatible with respect to polarity, such as the polyamide supports introduced by Sheppard and co-workers (Atherton, E. et al. (1975) J. Am. Chem. Soc. 97, 6584). These polyamide supports in conjunction with the use of polar aprotic solvents increased both yields and purity of peptide. Other workers looked to overcome the hydrophobicity of the polystyrene support by grafting a high molecular mass polyethylene glycol (PEG) chain onto a polystyrene resin. PEG is amphiphilic in nature, possessing both polar and non-polar characteristics. This amphiphilic nature not only provides the polar environment of the polyether backbone that assists the growing peptide chain, but has the additional benefit of engendering an ability of the resin to swell to a similar degree across a wide range of polar to non polar solvents, enabling its use in fixed reactor systems.

Polystyrene-polyethylene glycol (PS-PEG) graft copolymers have been prepared in one of two ways: (i) anionic polymerisation of ethylene oxide onto sites on the resin; or (ii) attachment of a preformed PEG onto an active site on the resin. An example of the former technique includes the first and possibly the best known PS-PEG hybrid resin, Tentagel^{™} developed by Bayer (Bayer E. (1991) Angew. Chem. Int. Ed. Engl. 30, 113-129, U.S. Patent No. 4,908,405 to Bayer). This matrix is prepared by coupling tetraethylene glycol (TEG) with chloromethylated polystyrene. A high molecular mass PEG is then introduced into the polymer by anionic graft polymerisation (reacting ethylene oxide with the potassium salt of PS-TEG). This gives rise to a polymer that possesses both the desired amphiphilic nature and the robustness of a polystyrene particle. ArgoGel™ is another resin prepared by anionic polymerization, in this case, a resin substituted with a diol to give a higher level of loading (Labadie et al., WO 97/27226). Both of these resins use anionic polymerisation of ethylene oxide and hence suffer from limitations of scalability of production due to safety considerations, as well as difficulties in controlling the batch to batch reproducibility of ethylene oxide loading, which makes them difficult and expensive to make.

PS-PEG resins that have been prepared via multi-step processes using preformed PEG derivatives and include PEGs linked by either amide or urethane linkages to PS as described by Barany (U.S. Patent No 5,545,698), and Champion™ and DendroGel™ resins described by Hudson and colleagues (Adams, J.H. et al. (1998) J. Org. Chem. 63, 3706-3716), respectively. These resins have been demonstrated to work equally well as the anionic polymerisation PS-PEG resins (Hudson, D. (1999) J. Comb. Chem. 1, 403-457).

Accordingly, there remains a need for an amphiphilic resin for efficient peptide synthesis from a one step reaction using readily available and low cost reagents and solid supports.

### SUMMARY OF THE INVENTION

Accordingly, it is a primary object of the invention to provide amphiphilic solid supports suitable for use in solid phase synthesis, and the like. It is a further object of the invention to provide methods of preparing amphiphilic solid supports. Accordingly, there is provided an amphiphilic solid support for solid phase synthesis, comprising a solid support and a compound according to Formula I covalently bound thereto: 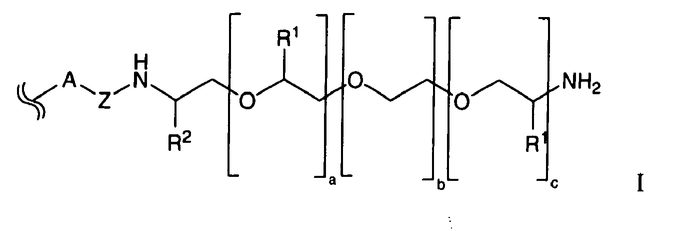 wherein A is C₅₋₁₀ aryl or heteroaryl, Z is either lower alkyl or absent, R¹ is lower alkyl, R² is H or lower alkyl, a is an integer from about 0 to about 20, b is an integer from about 5 to about 150, c is an integer from about 0 to about 20. In a particular embodiment, an amphiphilic solid support is provided, wherein a is from 0 to about 10, b is an integer from about 5 to about 100, c is from 0 to about 10, R¹ is methyl or ethyl and R² is methyl or ethyl. In another embodiment, an amphiphilic solid support is provided, wherein a is an integer from 0 to about 5, b is an integer between 5 and 60, c is an integer from 0 to about 5. In a preferred embodiment, an amphiphilic solid support is provided, wherein b is an integer from about 35 to about 45 and the sum of a + c is an integer between 4 and 8. In another preferred embodiment, an amphiphilic solid support is provided, wherein b is an integer from about 10 to about 15 and the sum of a + c is an integer between 4 and 8. In yet another preferred embodiment, an amphiphilic solid support is provided, wherein b is an integer from about 7 to about 11 and the sum of a + c is an integer between 2 and 6.

Preferably, the amphiphilic solid supports comprise a polymeric matrix prepared from crosslinked chloromethyl functionalized polystyrene/divinylbenzene, comprising 0.1% to 10% by weight divinylbenzene relative to styrene. The amphiphilic solid support is preferably in the form of beads, particles, membranes, or woven or nonwoven fibers.

Preferably, the amphiphilic solid supports provide an amine loading from about 0.01 to about 2 mmol/g dry weight of amphiphilic solid support.

In a preferred embodiment, an amphiphilic solid support is provided having a structure according to Formula II: 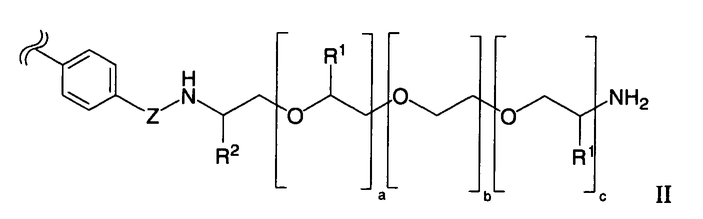 wherein Z is either lower alkyl or absent, R¹ is lower alkyl, R² is H or lower alkyl, a is an integer from 0 to about 20, b is an integer between 5 and 150, c is an integer from 0 to about 20. In yet other embodiments, methods of preparing amphiphilic solid supports for solid phase synthesis are provided, wherein the methods comprise providing a functionalized solid support, and covalently bonding thereto a compound according to Formula III: 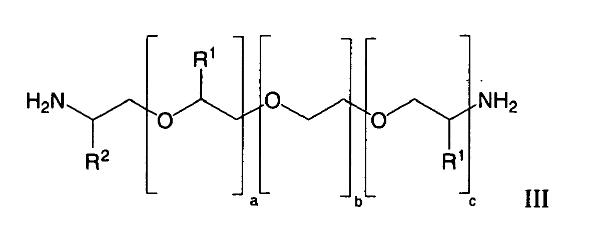 wherein R¹ is lower alkyl, R² is H or lower alkyl, a is an integer from 0 to about 20, b is an integer from about 5 to about 150, c is an integer from about 0 to about 20, wherein said covalently bonding forms a secondary amine linking the solid support and the compound of Formula III. In preferred embodiments, a is from 0 to about 10, b is from about 5 to about 100, c is from 0 to about 10, and R¹ is methyl or ethyl. In additional embodiments, b is from about 35 to about 45 and the sum of a + c is an integer between 4 and 8. In certain other embodiments, b is from about 10 to about 15 and the sum of a + c is an integer between 4 and 8. In certain additional embodiments, b is an integer from about 7 to about 11 and the sum of a + c is an integer between 2 and 6.

Preferably, the solid support comprises a material prepared from crosslinked chloromethyl functionalized polystyrene, crosslinked poly(meth)acrylates, or crosslinked poly(meth)acrylamides. In preferred embodiments, the amphiphilic solid supports comprise a polymeric matrix selected from crosslinked chloromethyl functionalized polystyrene/divinylbenzene, comprising 0.1% to 10% by weight divinylbenzene relative to the total weight of the polymer. In certain preferred embodiments, the functionalized solid support comprises a copolymer of chloromethyl styrene, styrene and divinylbenzene.
Preferably, the amphiphilic solid supports are in the form of particles with a mean diameter in the range between 0.1µm and 1000µm. In particular embodiments, the particles have a mean diameter in the range between 2µm and 500µm. In additional embodiments, the particles have a mean diameter in the range between 30µm and 300µm. In yet additional embodiments, the particles have an essentially monodisperse distribution and a mean diameter in the range between 2µm and 300µm.

Preferably, the amine loading is from about 0.01 to about 2 mmol/g dry weight of amphiphilic solid support.

In certain preferred methods, the functionalized solid support comprises a particle with a mean diameter between 30µm and 300µm formed from a copolymer of chloromethyl styrene, styrene and divinylbenzene to which is covalently bonded a polyoxyethylenediamine. Preferably the polyoxyethylenediamine is selected from Jeffamine-ED600, Jeffamine-ED900, or Jeffamine-ED2003 (Huntsman Corp., The Woodlands, TX).

Additional objects, advantages and novel features of the invention will be set forth in part in the description which follows, and in part will become apparent to those skilled in the art upon examination of the following, or may be learned by practice of the invention.

### BRIEF DESCRIPTION OF THE DRAWINGS

FIGS. 1A and B show a schematic of synthetic pathways to utilize various embodiments of amphiphilic solid supports of the invention for solid phase synthesis of peptides.

FIG. 2 illustrates a chromatogram showing the purity of a peptide prepared using three different peptide synthesis supports.

FIG. 3 illustrates a chromatogram showing the purity of a peptide prepared using three different peptide synthesis supports.

FIG. 4 illustrates a chromatogram showing the purity of a peptide prepared using three different peptide synthesis supports.

FIG. 5 illustrates a chromatogram showing the purity of a peptide prepared using three different peptide synthesis supports.

FIG. 6 illustrates a chromatogram showing the purity of a peptide prepared using three different peptide synthesis supports.

FIG. 7 illustrates a chromatogram showing the purity of a peptide prepared using a peptide synthesis support according to one embodiment of the invention.

FIG. 8 illustrates a chromatogram showing the purity of peptides prepared using peptide synthesis supports according to two embodiments of the invention.

### DETAILED DESCRIPTION OF THE INVENTION

### I. Definitions and overview

Before the present invention is described in detail, it is to be understood that unless otherwise indicated this invention is not limited to specific peptides, substrates, or the like, as such may vary. It is also to be understood that the terminology used herein is for the purpose of describing particular embodiments only and is not intended to limit the scope of the present invention.

It must be noted that as used herein and in the claims, the singular forms "a," "and" and "the" include plural referents unless the context clearly dictates otherwise. Thus, for example, reference to "amino acid" includes two or more amino acids; reference to "a polymer" includes two or more polymers, and so forth.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

As used herein the term "amphiphilic" refers to the property of possessing both hydrophilicity and hydrophobicity, resulting in wettability in a range of solvents and consistency of swelling in different solvents.

As used herein, the terms "element" and "monomeric unit" are used interchangeably to indicate components of the compound to be synthesized when the amphiphilic solid supports of the present invention are used for solid phase synthesis.

As used herein, the term "functionalized solid support" refers to the capability of the solid support to react with the amino moieties of polyoxyethylenediamines to yield secondary amines.

As used herein, the term "inert" indicates that the solid support to which the amphiphilic material is covalently bonded is sufficiently stable under conditions of use (e.g., reaction and washing steps using solvents, acids, bases, salts or buffers, etc.) such that the solid support can be used without degradation or leaching of materials off the support.

As used herein, the term "lower alkyl" refers to a branched or straight chain C₁₋₁₀ alkyl moiety.

As used herein, the terms "resin loading" or "functional loading" or "loading of solid support" refer to the number of chemical functional groups per weight of resin, typically expressed in units of millimoles (mmol) per gram.

As used herein, the terms "solid support," "substrate," or "resin" may be used interchangeably, and refer to a solvent insoluble material that is inert to washing or reaction conditions, such as those encountered during synthetic procedures utilized during solid phase synthesis, including solvent extraction steps and *in situ* reaction steps.

This invention pertains to amphiphilic solid supports for solid phase synthesis, as well as methods of making and using the amphiphilic solid supports to synthesize small molecule, oligomeric or polymeric materials such as peptides via solid phase peptide synthesis techniques. The present inventors have surprisingly discovered that an amphiphilic resin having improved synthesis efficiency can be provided using a one step reaction using readily available and low cost reagents and base solid support resin. This new amphiphilic resin contains both primary and secondary amine active sites which were surprisingly found by the inventors to possess similar reactivities with respect to peptide synthesis when coupled to the relevant linker. The presence of both primary and secondary amines also has the additional benefit of affording a higher resin loading than would be the case were only the primary amine active sites utilized, as in the case of other PS-PEG composite resins. The amphiphilic character and functional loading can be reliably controlled by the loading of the solid support and molecular mass of the amphiphilic component added. Additionally the process used to prepare this product is readily and safely scalable making the process and product more economical and amenable to larger-scale industrial applications.

Although practitioners have been extensively employing similar devices and methods for solid phase synthesis and the like, the present inventors are the first to discover an efficient and convenient method for covalently bonding an amphiphilic material to a solid support for solid phase synthesis, free of side reactions and toxic reactants, an unexpected and surprising result. The present inventors have further discovered that the present amphiphilic solid support provides a superior milieu for synthesizing peptides, providing superior and unexpected yields and synthetic efficiencies as well as superior products, free of contaminating side products.

Various aspects and embodiments of the invention will be described in greater detail below.

### II. Amphiphilic solid supports for solid phase synthesis

Amphiphilic solid supports for solid phase synthesis are provided, comprising a solid support and a compound according to Formula I covalently bound thereto: 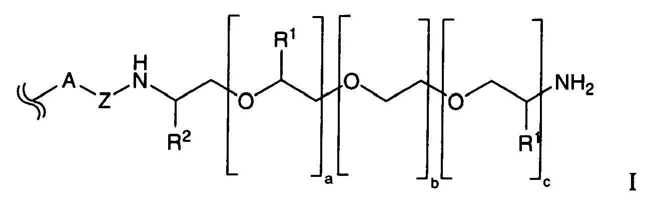 wherein A is C₅₋₁₀ aryl or heteroaryl, Z is either lower alkyl or absent, R¹ is lower alkyl, R² is H or lower alkyl, a is an integer from about 0 to about 20, b is an integer from about 5 to about 150, c is an integer from about 0 to about 20. In a particular embodiment, an amphiphilic solid support is provided, wherein a is from 0 to about 10, b is an integer from about 5 to about 100, c is from 0 to about 10, R¹ is methyl or ethyl and R² is methyl or ethyl. In another embodiment, an amphiphilic solid support s provided, wherein a is an integer from 0 to about 5, b is an integer between 5 and 60, c is an integer from 0 to about 5. In a preferred embodiment, an amphiphilic solid support is provided, wherein b is an integer from about 35 to about 45 and the sum of a + c is an integer between 4 and 8. In another preferred embodiment, an amphiphilic solid support is provided, wherein b is an integer from about 10 to about 15 and the sum of a + c is an integer between 4 and 8. In yet another preferred embodiment, an amphiphilic solid support is provided, wherein b is an integer from about 7 to about 11 and the sum of a + c is an integer between 2 and 6.

Preferably, the amphiphilic solid supports comprise a polymeric matrix prepared from crosslinked chloromethyl functionalized polystyrene/divinylbenzene, comprising 0.1% to 10% by weight divinylbenzene relative to the total weight of the polymer. The amphiphilic solid support is preferably in the form of beads, particles, membranes, or woven or nonwoven fibers.

Preferably, the amphiphilic solid supports provide an amine loading from about 0.01 to about 2 mmol/g dry weight of amphiphilic solid support.

In a preferred embodiment, an amphiphilic solid support is provided having a structure according to Formula II: 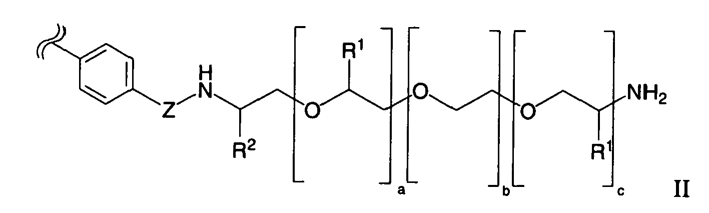 wherein Z is either lower alkyl or absent, R¹ is lower alkyl, R² is H or lower alkyl, a is an integer from 0 to about 20, b is an integer between 5 and 150, c is an integer from 0 to about 20.

### A. Solid Supports

The solid support can be formed from organic materials such as carbon fibers, cellulosic materials (e.g., nitrocellulose, cellulose acetate), synthetic polymers including poly(vinylchlorides), polyacrylamides, polyacrylates, polyolefins (e.g., polyethylene, polypropylene, polytetrafluoroethylene (PTFE)), poly(4-methylbutene), polystyrenes, polyurethanes, polyacrylonitriles, polymethacrylates, polymethacrylamides, poly(ethylene terephthalate), polysiloxanes, nylon, poly(vinyl butyrates), and the like, or mixtures or composites of any of the above. Preferably, a representative, but non-limiting, list of polymers that can be utilized in the solid support includes, but is not limited to, crosslinked methacrylates, crosslinked methacrylamides, crosslinked polystyrenes, poly(styrene divinylbenzene), copolymers comprising styrene or divinylbenzene and halogenated, alkylated or haloalkylated styrenes, pyridines, thiophenes, furans, imidazoles, and the like. Preferably, the solid support is functionalized so that it is capable of reacting with an amino moiety of an amphiphilic compound to yield a secondary amine.

The solid support preferably comprises a polymeric matrix comprising aryl or heteroaryl moieties, for example, poly(styrene divinylbenzene), copolymers of styrene or divinylbenzene with functionalized aryl or heteroaryl moieties such as styrenes or heterocycles carrying substituents such as halo or haloalkyl. Thus, a representative, but non-limiting, list of polymers that can be utilized in the solid support includes, but is not limited to, polystyrene, poly(styrene divinylbenzene), copolymers comprising styrene or divinylbenzene and halogenated or alkylated styrenes, pyridines, thiophenes, furans, imidazoles, and the like. In certain embodiments, the solid support can comprise a crosslinked polymeric matrix comprising aryl or heteroaryl moieties, for example, functionalized poly(styrene divinylbenzene), or copolymers of styrene or divinylbenzene with functionalized aryl or heteroaryl moieties such as styrenes or heterocycles carrying substituents such as halo or haloalkyl. In certain preferred embodiments, the functionalized solid support comprises a copolymer of chloromethyl styrene, styrene and divinylbenzene.

In more preferred embodiments, the amphiphilic solid supports comprise a polymeric matrix prepared from crosslinked chloromethyl functionalized polystyrene/divinylbenzene, comprising 0.1 % to 10% by weight divinylbenzene relative to the total weight of polymer.

In certain embodiments, the solid support can be porous, such as in the form of a polymeric bead, membrane or filter. The solid support can be in the form of a monolith, agglomerated particles, or woven or nonwoven fibers, preferably nonwoven fibers including macro and microfibrous webs such as melt-blown webs, spunbonded or air laid webs and blown fibrous webs, such as described in U.S. Patent No. 5,328,758 and references cited therein. Preferably, the solid support is in the form of a predominantly spherical bead or particle with mean diameters in the range between 0.1µm and 1000µm. In preferred embodiments, the particle has a mean diameter between 2µm and 500µm, or in other embodiments, between 30µm and 300µm. In another preferred embodiment, the particle has a mean diameter in the range of 2-300µm and is suitable for typical peptide or nucleotide synthesis apparatuses.

Preferably, the solid support is sufficiently inert to be used to prepare polypeptides, polynucleotides, polysaccharides, peptide-nucleic acids, chemical libraries, and the like, for example, involving the use of strong acids or base, without losing structural integrity. Preferably, the solid support is in a form that is suitable for performing solid phase synthesis.

### B. Amphiphilic compounds

The amphiphilic solid supports provided comprise a functionalized solid support, and covalently bound thereto a polyoxyethylenediamine compound according to Formula III: 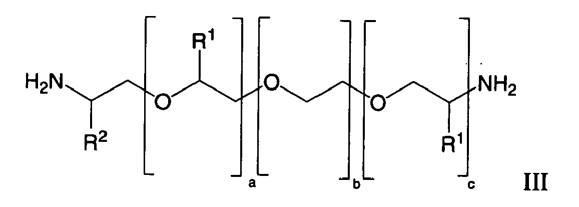 wherein R¹ is lower alkyl, R² is H or lower alkyl, a is an integer from 0 to about 20, b is an integer from about 5 to about 150, c is an integer from about 0 to about 20, wherein said covalent bonding forms a secondary amine linking the solid support and the compound of Formula III. In preferred embodiments, a is from about 0 to about 10, b is from about 5 to about 100, c is from 0 to about 10, and R¹ is methyl or ethyl. In additional embodiments, a is from about 0 to about 10, b is an integer from about 5 to about 100, c is from 0 to about 10, and R¹ is methyl or ethyl. In yet other embodiments, b is from about 35 to about 45 and the sum of a + c is an integer between 4 and 8. In certain other embodiments, b is from about 10 to about 15 and the sum of a + c is an integer between 4 and 8. In certain additional embodiments, b is an integer from about 7 to about 11 and the sum of a + c is an integer between 2 and 6.

Preferably, the amphiphilic compound is a polyoxyethylenediamine compound having a number average molecular weight between 200 and 6000, and more preferably, having a number average molecular weight of approximately 600, 900 or 2000.

In a particularly preferred embodiment, an O,O'-Bis (2-aminopropyl)propylene glycol-*block*-polyethylene glycol-*block*-propylene glycol is substituted directly onto a co-polymerised chloromethyl polystyrene bead (indicated by PS) giving rise to an amphiphilic solid support that possesses two reactive sites, the secondary amine linkage and the primary amine at the PEG terminus as shown below: 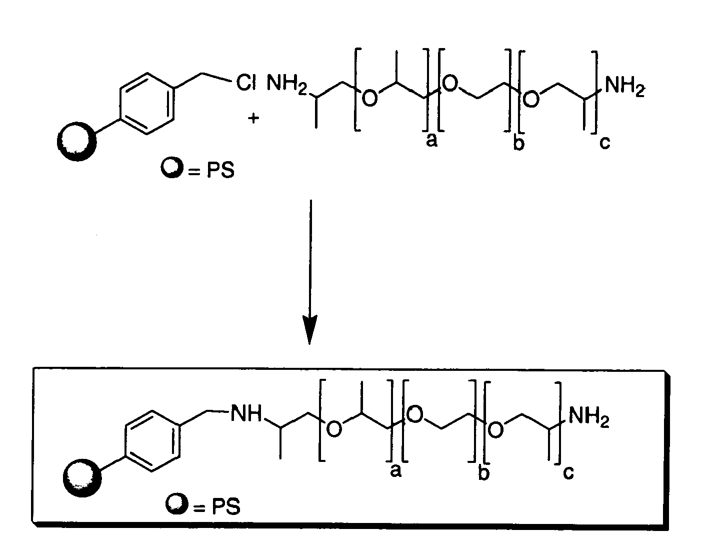 wherein a is an integer from about 0 to about 20, b is an integer from about 5 to about 150, c is an integer from about 0 to about 20. In a particular embodiments, a is from 0 to about 10, b is an integer from about 5 to about 100, c is from 0 to about 10, R¹ is methyl or ethyl and R² is methyl or ethyl. In another embodiment, a is an integer from 0 to about 5, b is an integer between 5 and 60, c is an integer from 0 to about 5. In a preferred embodiment, b is an integer from about 35 to about 45 and the sum of a + c is an integer between 4 and 8. In another preferred embodiment, b is an integer from about 10 to about 15 and the sum of a + c is an integer between 4 and 8. In yet another preferred embodiment, b is an integer from about 7 to about 11 and the sum of a + c is an integer between 2 and 6.

The two reactive amines make possible a PS-PEG resin with higher functional loading. Loading can be varied by using PEG diamines of different molecular weight, for example, using a range of commercially available PEG diamines having molecular weights from 200-6000 daltons (e.g., Jeffamine ED-series, Huntsman, The Woodlands, Texas).

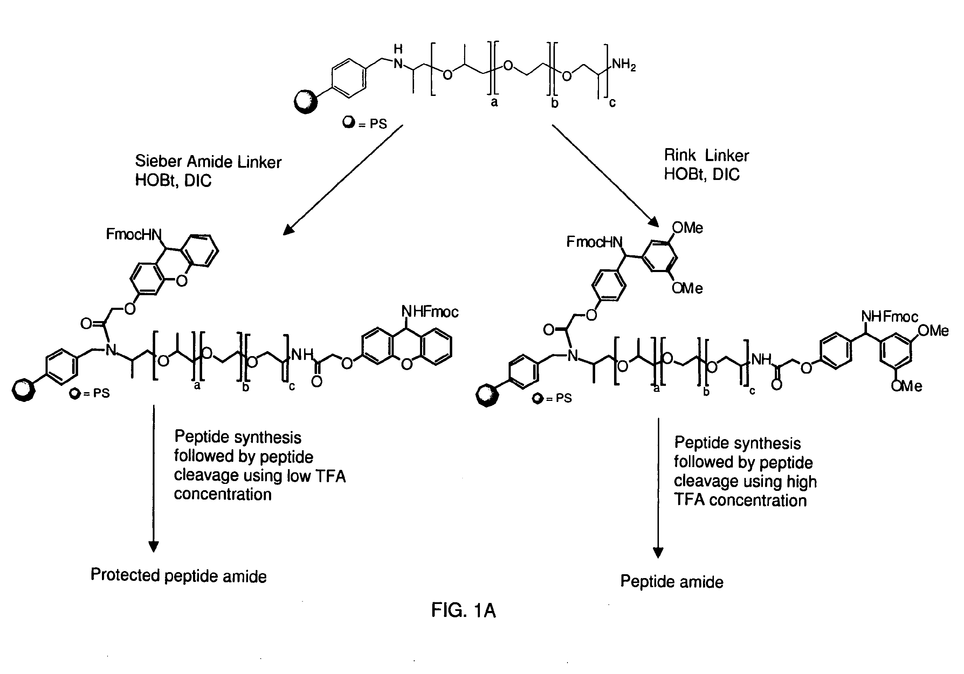

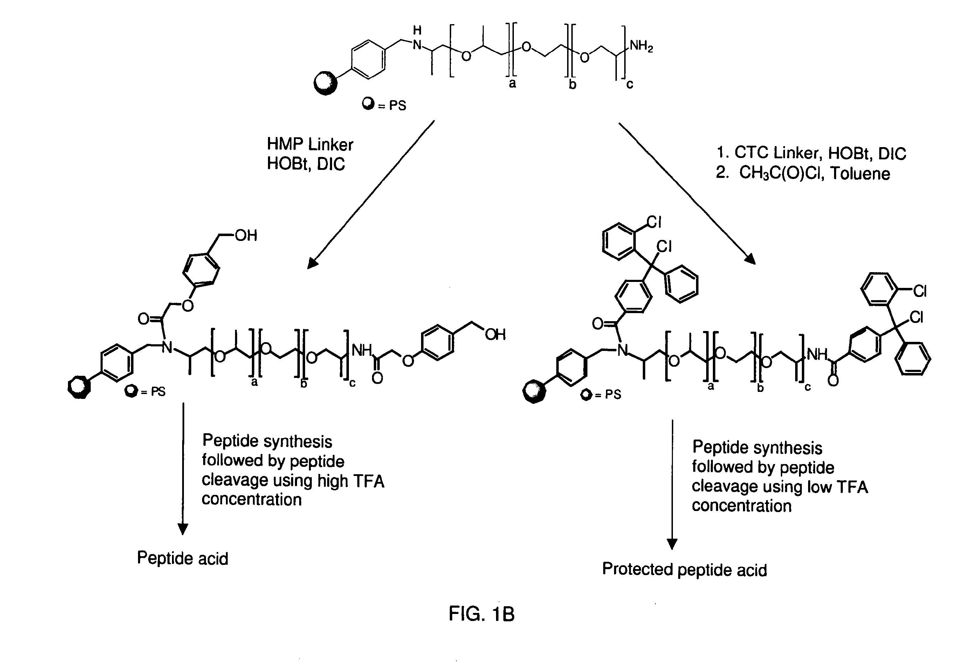

This new amphiphilic solid support is postulated to decrease the secondary structure effects that lead to sequence-dependent coupling deficiencies because of the hydrophilic environment provided by the PEG component of the solid support resin. It is postulated that the PEG chain is not acting as a spacer arm distancing the growing peptide from the hydrophobic polystyrene support, but rather it creates a favorable environment for peptide solvation, ameliorating aggregation effects due to peptide secondary structure (Adams, J.H. et al. (1998), J. Org. Chem. 63, 3706-3716). The two reactive sites on the ends of the linker, one next to the non-polar polystyrene and the other at the end of the more polar PEG chain, can surprisingly react equally well. A variety of linkers can be covalently bound to this support making possible the preparation of protected and deprotected peptide acids and peptide amides, of which the preparation of a few examples are shown in FIG. 1.

### III. Methods for preparing an amphiphilic solid support for solid phase synthesis

The invention further provides methods for preparing amphiphilic solid supports for solid phase synthesis, wherein the methods comprise providing a functionalized solid support, and covalently bonding thereto a compound according to Formula III: 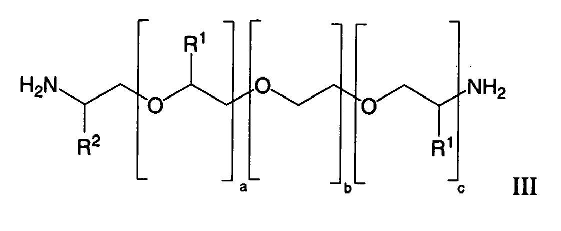 wherein R¹ is lower alkyl, R² is H or lower alkyl, a is an integer from 0 to about 20, b is an integer from about 5 to about 150, c is an integer from about 0 to about 20, wherein said covalent bonding forms a secondary amine linking the solid support and the compound of Formula III.

In preferred embodiments, a is from about 0 to about 10, b is from about 5 to about 100, c is from 0 to about 10, and R¹ is methyl or ethyl. In other embodiments, b is from about 35 to about 45 and the sum of a + c is an integer between 4 and 8. In certain other embodiments, b is from about 10 to about 15 and the sum of a + c is an integer between 4 and 8. In certain additional embodiments, b is an integer from about 7 to about 11 and the sum of a + c is an integer between 2 and 6.

Preferably, the solid support comprises a material selected from crosslinked functionalized polystyrene, crosslinked poly(meth)acrylates, or crosslinked poly(meth)acrylamides. In certain preferred embodiments, the functionalized solid support comprises a copolymer of chloromethyl styrene, styrene and divinylbenzene. In more preferred embodiments, the amphiphilic solid supports comprise a polymeric matrix prepared from crosslinked chloromethyl functionalized polystyrene/divinylbenzene, comprising 0.1% to 10% by weight divinylbenzene relative to the total weight of polymer.

Preferably, the amphiphilic solid supports are in the form of particles with a mean diameter in the range between 0.1µm and 1000µm. In particular embodiments, the particles have a mean diameter in the range between 2µm and 300µm. In additional embodiments, the particles have a mean diameter in the range between 30µm and 300µm. In yet additional embodiments, the particles have an essentially monodisperse distribution and a mean diameter in the range between 2µm and 300µm.

Preferably, the amine loading is from about 0.01 to about 2 mmol/g dry weight of amphiphilic solid support.

In certain preferred methods, the functionalized solid support comprises a particle with a mean diameter between 30µm and 300µm formed from a copolymer of chloromethyl styrene, styrene and divinylbenzene to which is covalently bonded a polyoxyethylenediamine.
Preferably the polyoxyethylenediamine is selected from Jeffamine-ED600, Jeffamine-ED900, or Jeffamine-ED2003 (Huntsman Corp., The Woodlands, TX).

In a preferred embodiment, the amphiphilic solid support can be prepared by reacting an appropriately functionalized solid support, such as a cross-linked polymer, with a polyoxyethylenediamine. Commonly, the appropriately functionalized crosslinked polymer is functionalized either directly or via a lower alkyl spacer with halo (e.g., chloride, bromide or iodide) or other suitable leaving group for nucleophilic substitution with the amino moiety of the polyoxyethylenediamine. The crosslinked polymer is typically a copolymer comprising phenylethylene and chloromethylphenylethylene units and can be prepared by polymerising styrene and chloromethylstyrene with divinylbenzene (DVB) as the crosslinking monomer. Although DVB is the preferred crosslinking monomer, other suitable crosslinking monomers include multifunctional (meth)acrylates, including di/triacrylates or di/trimethacrylates such as, without limitation, ethylene glycol diacrylate, ethylene glycol dimethacrylate, trimethylopropane trimethacrylate trivinyl benzene or N,N-bis acryloyl ethylene diamine, and the like. Preferably 0.5 to 5% by weight of crosslinking monomer is used.

Crosslinked polymers containing a chloride or other suitable leaving group are preferably produced as beads ranging in size from 0.1-1000µm. More preferably, the bead size is from 2 µm to 300 µm, or even more preferably from 30 µm to 300µm.

The cross-linked polymer beads are preferably produced by an aqueous suspension polymerisation process, as described, for example, by Ford (1982) J. Applied Polymer Science 27, 133-138. In this process, the monomers are suspended in an aqueous phase as droplets, most commonly by stirring, which are stabilised to prevent agglomeration to form larger droplets with the addition of a stabiliser to the aqueous phase. Typical stabilisers can be polyvinyl alcohol (PVA), cellulose based stabilisers, sodium dodecyl sulphate (SDS), etc.

Polymerisation is generally achieved using a free radical process. The free radicals are generated by the thermal decomposition of a free radical initiator. Typical initiators are peroxides (benzoyl, dicotyl, lauryl, and the like): initiators (AIBN) or other commonly used thermally decomposable molecules. Droplets in the suspension mixture are heated, typically between 50-90°C, to decompose the initiators, generating free radicals and leading to subsequent polymerisation. The resultant particles are filtered or centrifuged and washed with suitable solvents such as tetrahydrofuran, methanol, and water, and following drying, size classified by sieving.

### IV. Methods for using an amphiphilic solid support for solid phase synthesis

The use of solid phase synthesis resins, such as the amphiphilic solid supports of the present invention, first found popularity in the synthesis of oligomers such as peptides, oligonucleotides, peptide nucleic acids, and oligosaccharides, leading to great advancements in the preparation of these products. In more recent times the potential for these solid phase synthesis resins in synthetic organic chemistry was realised and their use extended to the synthesis of a myriad of different organic non oligomeric compounds in what is termed solid phase organic synthesis (SPOS). *See* Organic Synthesis on Solid Phase by F.Z. Dorwald, Wiley-VCH, Weinheim, FRG (2000) for general guidance on reactions. In SPOS, the ligand or synthon is attached to the solid phase support resin via a linker molecule, and subsequent coupling reactions are performed to synthesize the desired products. The linker molecule allows for clean and easy cleavage of the product from the solid support, when the on-resin reaction steps to form the desired target molecule are complete. SPOS and the development of combinatorial chemistry, whereby many thousands of compounds can be made in a single preparation by means of a technique of splitting and mixing, has led to the development of new polymer supports and libraries of chemical products. *See* Combinatorial Chemistry by W. Bannwarth and B. Hinzen, Wiley-VCH, Weinheim, FRG (2006) for guidance on reactions in combinatorial chemistry.

In solid phase peptide synthesis (SPPS), as in SPOS, the principles are similar in that the amino acid (or a peptide precursor) is attached to the solid phase support resin via a linker molecule that allows for subsequent on-resin reaction steps to form the desired target molecule, which then can be easily and cleanly cleaved from the solid support. This technique has an advantage over solution phase reactions in that on-resin transformations can be forced to completion using a large excess of reactants, which following reaction can be removed, along with side products, from the resin product by simple washing and filtration. Additionally, solid phase synthesis of molecules, such as peptides requiring a large amount of repetitive steps, lends itself to automation, greatly facilitating the efficiency and productivity of the synthesis. The same principles apply for oligo- and polynucleotide, oligo- and polysaccharide synthesis, and the like.

Of great importance to the peptide chemist from the outset of preparation of a target peptide is the consideration of the linker (or handle) with respect to the protection scheme used. The linker is a bifunctional molecule that provides a reversible linkage between the solid support and the synthetic peptide chain. Attachment of the peptide chain to the linker normally occurs via the C (carboxy) terminus of the first amino acid, which has temporary protection on the N (amino) functionality, and peptide elongation occurs in the C → N direction. The linker is stable to the conditions of N deprotection and subsequent coupling reactions during peptide elongation, but is easily and cleanly cleaved once the desired peptide sequence is assembled. Depending on the linker used, the peptide that is cleaved can be in the form of the free acid or a derivative thereof, such as amide or alkylamide. Additionally, depending on linker and cleavage conditions used, the peptide product can be obtained fully side chain protected or deprotected.

Briefly, peptide synthesis is performed using the following general procedures. Orthogonal side-chain protection of Fmoc-amino acids with respect to the linker is used - i.e. base stable under the conditions required for Fmoc- group cleavage whilst typically cleaved under acidic conditions required to release the synthesized peptide from the solid support. Examples of such protecting groups include: Boc-; tert-Bu-, Trityl, Pbf- (2,2,4,6,7-pentamethyldihydrobenzofuran-5-sulfonyl). The basic synthesis cycle generally involves an initial wash with a wash solvent (e.g., DMF, twice), followed by Fmoc cleavage with piperidine-DMF (for example, 3:7, 1 min + 10 min), and then washing with DMF (5 times), and coupling with the appropriate activated amino acid solution for the appropriate amount of time (typically 30 minutes to several. In the case of difficult sequences, multiple coupling steps are typically used to ensure complete reaction. After final Fmoc removal, the resins are washed with CH₂Cl₂ (typically 3 times), and MeOH (typically once) and dried. The resins can be cleaved with a variety of "cleavage cocktails" depending on the amino acid sequence prepared, typically TFA-triisopropylsilane-water (for example, 95:2.5:2.5) for 2 h could be used. The cleavage solutions are separated from the resin typically by filtration, collected in appropriate containers and concentrated by evaporation under reduce pressure to a small volume or oil. Precipitation by treatment with cold ether affords the peptide and assists in removal of residual "cleavage cocktail". Following separation from the ether supernatant the peptide is typically re-constituted in aqueous solution and lyophilized. The samples are then analyzed by analytical HPLC and MS, or other appropriate analytical technique before use.

The amphiphilic support described in the present invention comprises amino group functionalities, and there are many suitable linkers known in the art that can be attached to these amino groups. Commonly, the linkers possess a free carboxylic acid moiety that reacts with the amino group to form a stable amide linkage to the solid support. Linkers which can be attached to amino groups include, but are not limited to, Rink amide linkers (e.g.., p-[(R,S)-α-[1-(9H-fluoren-9-yl)-methoxyformamido]-2,4-dimethoxybenzyl]-phenoxyacetic acid); HMP linkers (e.g., 4-(hydroxymethyl)phenoxyacetic acid); Trityl linkers (e.g., 4-(diphenylhydroxymethyl) benzoic acid (a Trityl alcohol linker) and 4-[(2-chlorophenyl)hydroxyphenylmethyl]-benzoic acid (a 2-Chlorotrityl alcohol linker); Sieber linkers (e.g., 4-(9-Fmoc-aminoxanthen-3-yloxy)-butyric acid (Sieber amide, butyric acid linker) and 5-(9-Fmoc-aminoxanthen-3-yloxy)-pentanoic acid (Sieber-amide, valeric acid linker); Alkylamide inodole-acetic acid linkers (e.g., 3-(N-Fmoc-N-[R]-aminomethyl)-indol-1-yl)}-acetic acid, wherein [R] is lower alkyl). *See also* Solid-Phase Synthesis, A Practical Guide by S.A. Kates and F. Albericio, Marcel Dekker, Basel, (2000) for additional commonly used linkers.

Attachment of the linker molecule to the amine functionality of the support is typically effected by coupling reagents that activate the carboxylic acid. Coupling reagents generally include carbodiimides such as diisopropylcarbodiimide or iminium reagents such as *N*-[1*H-*benzotriazol-1-yl(dimethylamino)methylene]-N-methylamethanaminium hexafluorophosphate *N*-oxide (HBTU). These coupling reagents are also typically used in the peptide elongation reactions and more comprehensive information on coupling reagents used in peptide synthesis is given in Fmoc Solid Phase Peptide Synthesis by W.C. Chan and P. D. White published by Oxford University Press (2000).

In addition to the aforementioned solid phase synthesis reactions, the supports described in the present invention, have potential use in the areas of affinity and adsorption chromatography, enzyme immobilization (e.g., for use in bioreactors) and as polymer supported reagents, scavengers and catalysts (S. Ley et al. (2000) J. Chem Soc Perkin Trans. 1, 3815-4195).

Accordingly, in certain embodiments, methods are provided for using the amphiphilic solid supports of the invention for solid phase synthesis. In one embodiment, methods for performing solid phase synthesis are provided, said methods comprising: a) providing an amphiphilic solid support according to Formula I, wherein A is C₅₋₁₀ aryl or heteroaryl, Z is either lower alkyl or absent, R¹ is lower alkyl, R² is H or lower alkyl, a is an integer from about 0 to about 20, b is an integer from about 5 to about 150, c is an integer from about 0 to about 20, b) covalently coupling to at least one amine of the compound of Formula I a linker that enables attachment of a first element or monomeric unit (e.g., an amino acid, nucleotide or sugar) in a polymer to be synthesized, wherein said linker is suitable for performing solid phase synthesis of a small molecule, 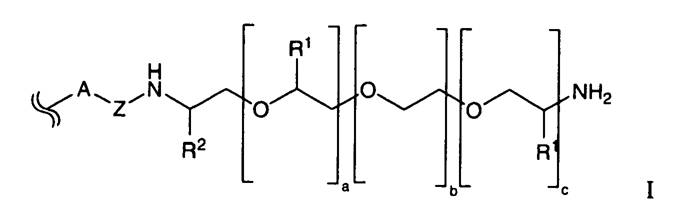 oligomeric or polymeric compound, or chemical library of compounds, c) covalently coupling to the linker an element or monomeric unit of the small molecule, oligomer or polymer, or library of compounds to be synthesized, d) repeating the coupling steps with additional elements or monomeric units until the desired products have been synthesized, and e) optionally cleaving the synthesized product from the amphiphilic solid support. Preferably, the linkers include, but are not limited to, Rink amide linkers (e.g.., p-[(R,S)-α-[1-(9H-fluoren-9-yl)-methoxyformamido]-2,4-dimethoxybenzyl]-phenoxyacetic acid); HMP linkers (e.g., 4-(hydroxymethyl)phenoxyacetic acid); Trityl linkers (e.g., 4-(diphenylhydroxymethyl) benzoic acid (a Trityl alcohol linker) and 4-[(2-chlorophenyl)hydroxyphenylmethyl]-benzoic acid (a 2-Chlorotrityl alcohol linker); Sieber linkers (e.g., 4-(9-Fmoc-aminoxanthen-3-yloxy)-butyric acid (Sieber amide, butyric acid linker) and 5-(9-Fmoc-aminoxanthen-3-yloxy)-pentanoic acid (Sieber-amide, valeric acid linker); Alkylamide indole-acetic acid linkers (e.g., 3-(N-Fmoc-N-[R]-aminomethyl)-indol-1-yl)}-acetic acid, wherein [R] is lower alkyl), and the like.

In certain embodiments, the linker can optionally comprise a first element or monomeric unit already covalently bound thereto to expedite the synthesis process. In this embodiment, the initial element or monomeric unit is covalently bound to the amphiphilic solid support through the linker, and additional coupling steps are performed to further synthesize the desired product, and cleave it from the solid support, as described above. For example, FIG 1A illustrates the steps for synthesizing a protected peptide amide or peptide amide using the amphiphilic solid supports of the invention with Sieber amide linkers and Rink amide linkers, respectively. In another example, FIG 1B illustrates the steps for synthesizing protected peptide acids or peptide acids using the amphiphilic solid supports of the invention with trityl linkers and HMP linkers, respectively.

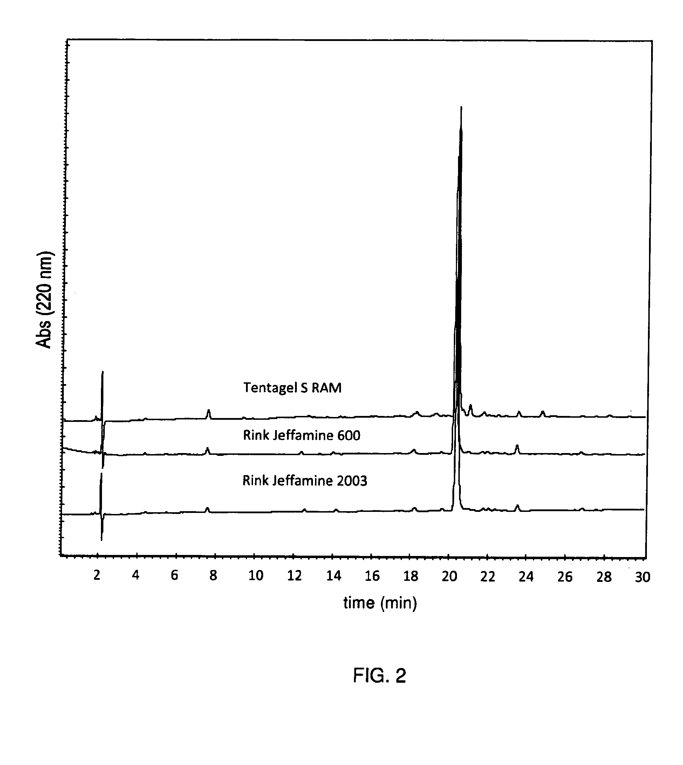

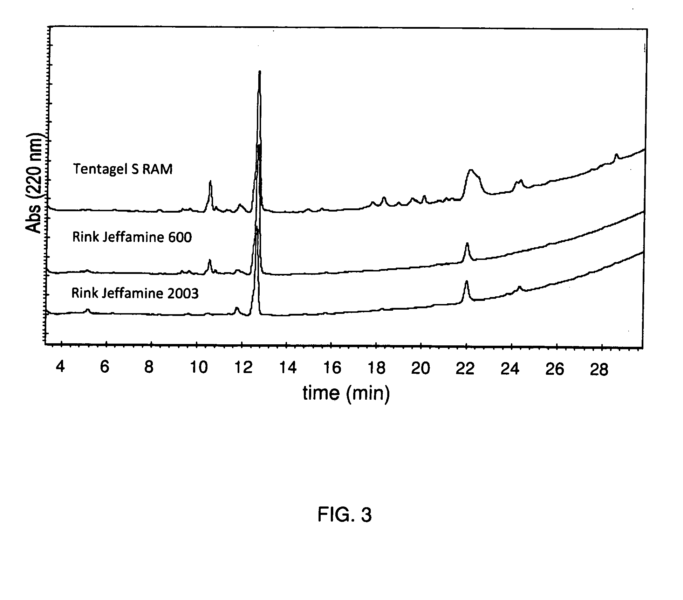

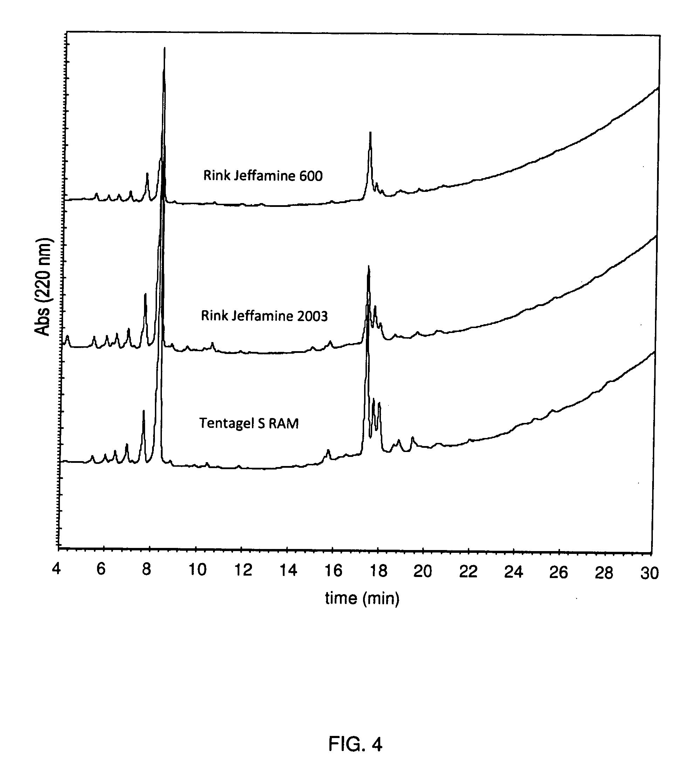

As described in Examples 7-10, the amphiphilic solid supports of the invention can be used with good results in the synthesis of peptides. In Example 7, three different peptides were synthesized using two exemplary Rink Amide polyoxyethylenediamine-modified resins (described in Example 3) and compared with the synthesis of the same peptides using a prior art solid phase synthesis resin (Rink Tentagel). As shown in FIG. 2 and summarized in Table 1, Acyl Carrier Protein (ACP) synthesized using the different resins tested showed similar chromatographic profiles, indicating the same peptide was synthesized in all cases. However, the yields of ACP using the polyoxyethylenediamine-modified resins were higher, and the purity by HPLC was higher. In addition, as shown in FIG. 3 and summarized in Table 1, while β-Amyloid Peptide Amide Fragment (34-42) synthesized using the different resins showed similar chromatographic profiles, the yields of β-Amyloid using the polyoxyethylenediamine-modified resins were much higher, and the purity by HPLC was higher, as significant contaminating side products are visible on the chromatogram of synthesized using Tentagel. Further, as shown in FIG. 4 and summarized in Table 1, while Deca(alanyl)valinamide synthesized using the different resins showed similar chromatographic profiles, the yields of Deca(alanyl)valinamide using the polyoxyethylenediamine-modified resins were greater, and the purity by HPLC was higher, as significant contaminating side products are visible on the chromatogram of the peptide produced using Tentagel.

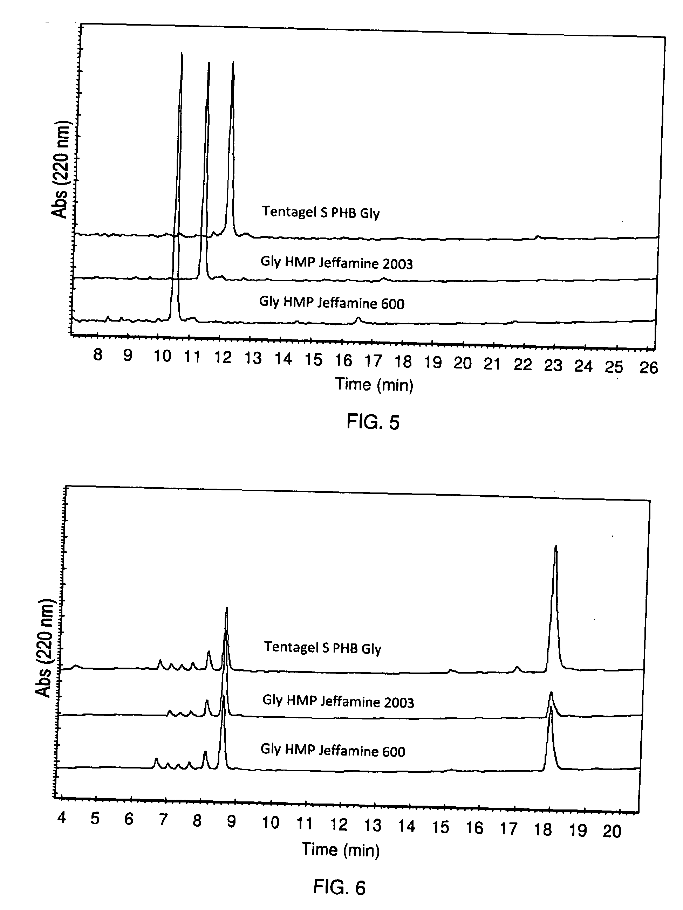

In Example 8, ACP was synthesized using two exemplary Fmoc-amino acid-HMP-polyoxyethylenediamine-modified resins (described in Example 4) and compared with the synthesis of the same peptides using a prior art solid phase synthesis resin (PHB-Tentagel). As shown in FIG.5 and summarized in Table 2, while ACP synthesized using the different resins showed similar chromatographic profiles (the different sample runs are shown offset for better visualization of detail), ACP yields using the polyoxyethylenediamine-modified resin (MW 600) was higher, and the purity by HPLC was higher for both resins compared with Tentagel. In addition, as shown in FIG. 6 and summarized in Table 2, while Deca(alanyl)valine synthesized using the different resins showed similar chromatographic profiles, the purity by HPLC was much higher, as significant contaminating side products are visible on the chromatogram, particularly for peptide synthesized using Tentagel.

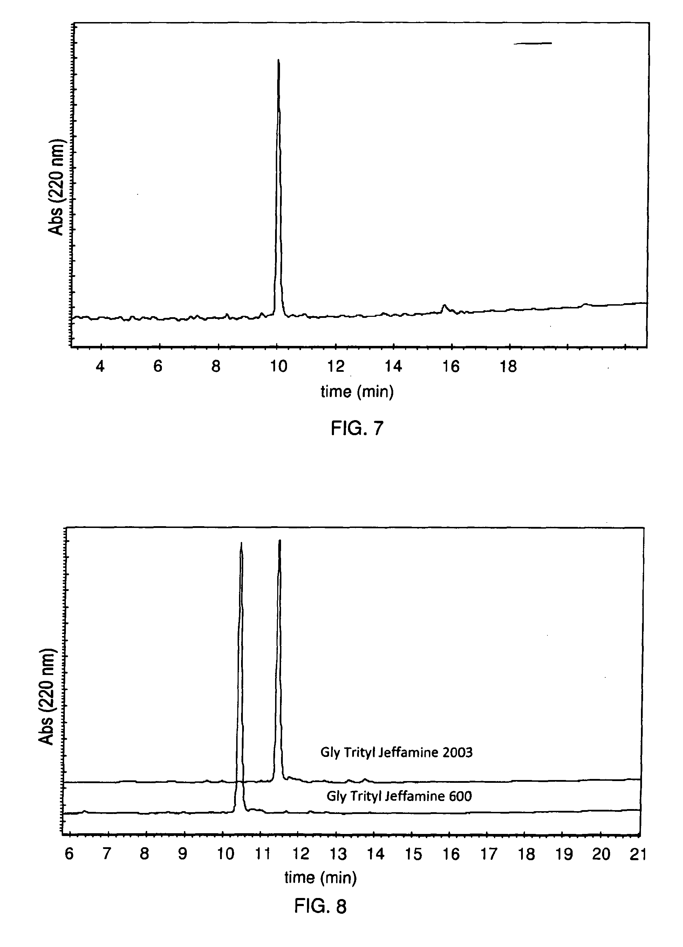

In Example 9, ACP was synthesized using the Sieber Amide polyoxyethylenediamine-modified resin (MW 2000). As shown in FIG. 7 and summarized in Table 3, ACP synthesized using Sieber Amide polyoxyethylenediamine-modified resin (MW 2000) showed a very clean chromatographic profile, with little evidence of contaminating deletion sequences, and a very good yield of 94%.

In Example 10, ACP was synthesized using Fmoc-Gly-Trityl polyoxyethylenediamine-modified resins (MW 600 and MW 2000). As shown in FIG. 8 and summarized in Table 4, ACP synthesized using the two different polyoxyethylenediamine-modified resins (MW 600 and MW 2000) showed similar chromatographic profiles (the different sample runs are shown offset for better visualization of detail). The chromatographic profiles were very clean, with little evidence of contaminating deletion sequences, and yields were very good at 90% and 93%, respectively.

### V. Applications and methods of use

The compositions and methods of the present invention can be advantageously used in the solid phase synthesis of any susceptible material known in the art. Typical materials that can be synthesized using solid phase synthesis techniques using the present amphiphilic solid support include, without limitation, peptides, peptide nucleic acids, oligo- and polynucleotides, oligo- and polysaccharides, peptidomimetics, compounds for chemical libraries, chemical intermediates, and the like. In preferred embodiments, the amphiphilic solid support is used in the preparation of peptides, peptide libraries, peptidomimetics, peptide nucleic acids, and the like.

### VI. Advantages of the invention

In prior art methods where a pre-formed PEG derivative is used, the base resin used is a relatively expensive amino functionalised polystyrene, a product that is generally derived from a multiple step process (as described, for example, in U.S. Patent No 5,545,698 to Barany, and by Adams, J.H. et al. (1998) J. Org. Chem. 63, 3706-3716). Additionally, in most cases the prior art PS-PEG resins are prepared via several steps involving a derivatized PEG component that needs further treatment to liberate the active group. These additional steps incur an additional cost, making the final products expensive. Prior art methods using direct attachment of preformed unmodified PEG onto a chloromethylated polystyrene resin suffer from additional deficiencies, for example, crosslinking may occur whereby both terminal hydroxy groups of polyoxyethylene react with the chloromethylated polystyrene. In this event, the useful functional loading is decreased, resulting in decreased sites for covalent attachment of linkers and other reactants.

In contrast, the present amphiphilic solid supports and methods for their preparation overcome these deficiencies of the prior art solid supports. The present amphiphilic supports provide increased functional loading, and hence increased capacity of solid phase synthesis. In addition, the present methods of preparation provide superior loading yields using a one step reaction from the functionalized support, without requiring the use of more expensive base support materials (e.g. aminomethyl polystyrene) or complicated or hazardous reaction conditions. The amphiphilic character and functional loading can be reliably controlled by the amount of the functionalized monomeric unit in the polymeric matrix (e.g., chloromethylstyrene) and molecular mass of PEG component added. Additionally the process used to prepare this product is readily and safely scalable making the process and product more economical and amenable to larger-scale industrial applications. The resulting amphiphilic solid supports provide a superior milieu for synthesizing peptides and other synthetic reactions, providing superior and unexpected yields and synthetic efficiencies as well as superior products, free of contaminating side products.

Accordingly, the inventive compositions and methods are a significant advance over the prior art in efficiency of preparation and use, and provide economic and environmental benefits.

In the following examples, efforts have been made to ensure accuracy with respect to numbers used (e.g., amounts, temperature, etc.) but some experimental error and deviation should be accounted for. Unless indicated otherwise, temperature is in degrees °C and pressure is at or near atmospheric. All solvents were purchased as HPLC grade, and all reactions were routinely conducted under inert gas atmospheres unless otherwise indicated. All peptides synthesized in the examples were analyzed by LC-MS to confirm correct molecular mass of product using a Waters LCT mass spectrometer.

### Abbreviations:

- ACN: acetonitrile
- ACP: Acyl Carrier Peptide
- BOC: t-Butyloxycarbonyl
- DCM: dichloromethane
- DIPEA: *N, N*-Diisopropylethylamine
- DIC: *N, N*-Diisopropylcarbodiimide
- DMF: *N, N*-dimethylformadide
- EDT: Ethanedithiol
- Fmoc: 9-Fluorenylmethyloxycarbonyl
- HBTU: *N*-[1*H*-benzotriazol-1-yl(dimethylamino)methylene]-*N*-methylamethanaminium hexafluorophosphate *N*-oxide
- HMP: 4-(Hydroxymethyl)phenoxyacetamide
- HOBt: 1-Hydroxybenzotriazole
- MeOH: Methanol
- NMP: *N*-Methylpyrolidinone
- PHB: p-Hydroxybenzyl
- TC: trityl chloride
- TFA: trifluoroacetic acid
- TIPS: Triisopropylsilane

### Example 1

### Preparation of Supported Polyoxyethylenediamine (MW 2000)

To a stirred solution of Jeffamine ED 2003 (Huntsman Corporation, The Woodlands, Texas)(179g, 90mmol) dissolved in N,N'-dimethylacetamide (150mL) and N,N'-diisopropylethylamine (31mL), the 4-chloromethylstyrene resin (15g, 0.6 mmole/g, 9mmol) was added, and the resultant mixture stirred at 60°C for 20 h. The polyoxyethylenediamine-modified resin was then filtered and washed successively with DMF, MeOH, DMF, DCM and MeOH before drying under vacuum to yield 25.5g of product. A resin loading value of 0.52mmol N /g was calculated from elemental carbon, hydrogen and nitrogen analysis.

### Example 2

### Preparation of Supported Polyoxyethylenediamine (MW 600)

To a stirred solution of Jeffamine ED 600 (Huntsman Corporation, The Woodlands, Texas)(360g, 600mmol) dissolved in N,N'-dimethylacetamide (1L) and N,N'-diisopropylethylamine (208mL), the 4-chloromethylstyrene resin (100g, 60mmol) was added, and the resultant mixture stirred at 60° C for 20 h. The polyoxyethylenediamine-modified resin was then filtered and washed successively with DMF, MeOH, DMF, DCM and MeOH before drying under vacuum to yield 133.8g of product. A resin loading value of 0.84mmol N /g was calculated from elemental carbon, hydrogen and nitrogen analysis.

### Example 3

### Preparation of Rink Amide Polyoxyethylenediamine-Modified Resins

To the polyoxyethylenediamine-modified resins prepared in Examples 1 and 2 (1 mol equiv) swollen in DMF/DCM 2:1 (12ml/g resin), Fmoc-Rink linker ( p-[(R,S)-α-[1-(9H-Fluoren-9-yl)-methoxyformamido]-2,4-dimethoxybenzyl]-phenoxyacetic acid, Novabiochem ) (2 mol equivalent with respect to resin), 1-Hydroxybenzotriazole, (2 mol equivalent with respect to resin), and diisopropylcarbodiimide (4 mol equivalent with respect to resin). The reaction mixture was shaken for 20h following which the resin was filtered and washed successively with DMF, DMF/pyridine /acetic anhydride , DMF, MeOH, DMF, DCM and MeOH before drying under vacuum to yield Rink amide polyoxyethylenediamine-modified resin. An Fmoc loading assay was used to determine the level of loading for each resin, as described by Gude et al. (2002) Letters in Peptide Science, 9, 203. The Rink amide polyoxyethylenediamine (MW 600)-modified resin gave a loading of 0.61mmol/g and the Rink amide polyoxyethylenediamine (MW 2000)-modified resin gave a loading of 0.41mmol/g.

### Example 4

### Preparation of 4-(Hydroxymethyl)phenoxyacetamide (HMP)- and Fmoc-amino acid-HMP-Polyoxyethylenediamine-Modified Resins

To the polyoxyethylenediamine-modified resins prepared in Examples 1 and 2 (1 mol equiv) swollen in DMF/DCM 2:1 (12ml/g resin), HMP linker (2 mol equiv with respect to resin), 1-Hydroxybenzotriazole, (2 mol equiv with respect to resin), and diisopropylcarbodiimide (4 mol equiv with respect to resin) were added. The reaction mixture was shaken for 20h following which the resin was filtered and washed successively with DMF, MeOH, DMF, DCM and MeOH before drying under vacuum to yield HMP-polyoxyethylenediamine-modified resins.

The HMP- polyoxyethylenediamine-modified resin was functionalised with Fmoc-amino acids as follows: to the resin swollen in DMF (8 mL/g resin), the Fmoc-amino acid (5 mol equiv with respect to resin) and pyridine (8.3 mol equiv with respect to resin) were added followed by addition of 2,6-dichlorobenzoyl chloride (5 mol equiv with respect to resin). The mixture was shaken for 18h following which the resin was filtered off and washed with DMF, MeOH, DMF, DCM and MeOH before drying under vacuum at room temperature. An Fmoc loading assay was used to determine the level of loading of each resin; the results of which are as follows: Fmoc-Gly-HMP polyoxyethylenediamine-modified resin (MW 600), 0.59 mmol/g; Fmoc-Gly-HMP polyoxyethylenediamine-modified resin (MW 2000) 0.36 mmol/g; Fmoc-Val-HMP polyoxyethylenediamine-modified resin (MW 600) 0.49 mmol/g; Fmoc-Val-HMP polyoxyethylenediamine-modified resin (MW 2000) 0.25 mmol/g. It can be seen that the loading decreases with increasing molecular mass of polyoxyethylenediamine modification of the resin support.

### Example 5

### Preparation of Tritylchloride (TC) Polyoxyethylenediamine-Modified Resins

To the polyoxyethylenediamine-modified resins prepared in Examples 1 and 2 (1 mol equiv) swollen in DMF/DCM 2:1 (12 ml/g resin), 4-(diphenylhydroxymethyl) benzoic acid (2 mol equiv with respect to resin), 1-hydroxybenzotriazole, (2 mol equiv with respect to resin), and diisopropylcarbodiimide (4 mol equiv with respect to resin) were added. The reaction mixture was shaken for 20h following which the resin was filtered and washed with DMF, MeOH, DMF, DCM and MeOH before drying under vacuum to yield the intermediate trityl alcohol-modified resin. This trityl alcohol-modified resin (0.5g) was swollen in toluene and acetyl chloride (0.43 mL) was added. The mixture was heated to 60°C with stirring under inert gas overnight. The resin was washed with toluene and DCM (5 times each solvent) before being treated with a solution of Fmoc-Glycine (2 mol equiv with respect to resin) in DCM (3mL) and DIPEA (4 mol equiv with respect to resin). Following 2h shaking, the resin was filtered and washed with DCM/MeOH/DIPEA 17:2:1 (v/v/v), DCM, DMF, DCM, MeOH (3 times with each solvent) before drying under vacuum at room temperature. An Fmoc loading assay was used to determine the level of loading of each resin. The TC polyoxyethylenediamine-modified resin (MW 600) gave a loading of 0.48 mmol/g and the TC polyoxyethylenediamine-modified resin (MW 2000) gave a loading of 0.29 mmol/g.

### Example 6

### Preparation of Sieber Amide Polyoxyethylenediamine-Modified Resins

To the polyoxyethylenediamine-modified resins prepared in Examples 1 and 2 (1 mol equiv) swollen in DMF/DCM 2:1 (12 ml/g resin), 4-(9-Fmoc-aminoxanthen-3-yloxy)-butyric acid (2 mol equiv with respect to resin), 1-Hydroxybenzotriazole, (2 mol equiv with respect to resin), and diisopropylcarbodiimide (4 mol equiv with resin) were added. The reaction mixture was shaken for 20h following which the resin was filtered and washed with DMF, MeOH, DMF, DCM and MeOH before drying under vacuum to yield Sieber amide modified resin. An Fmoc loading assay was used to determine the level of loading of each resin. The Sieber polyoxyethylenediamine-modified resin (MW 600) gave a loading of 0.52mmol/g and the Sieber polyoxyethylenediamine-modified resin (MW 2000) gave a loading of 0.29mmol/g.

### Example 7

### Comparative Synthesis of Peptide Amides Using Rink Polyoxyethylenediamine-modified Resins and Rink Tentagel Resin

Peptide amides to be synthesized were as follows:
a. VAQAAIDYING (Acyl Carrier Protein (ACP) (amino acids 65-74))
b. LMVGGVVIA (B Amyloid fragment (amino acids 34-42))
c. AAAAAAAAAAV (Deca(alanyl)valinamide)
   The peptide amides were synthesized on a 0.1mmol scale (except for the first entry in Table 1 which was performed by a commercial custom peptide service on a 0.2 mmol scale using double couplings, and syntheses using Rink polyoxyethylenediamine modified resin (MW 600) where a 0.084 mmol scale was used) using the FastMoc methodology on an Applied Biosystems 431A instrument using 10 equivalents of the reagents and Fmoc-amino acids in NMP for coupling reactions. HBTU/HOBt were used as the coupling reagents in the Fastmoc cycles and deprotection steps done with DMF/piperidine 20:80 v/v. Only single Fmoc amino acid couplings were used. Support resins used were: Tentagel S RAM (Rapp Polymere GmbH, Tubingen, 0.26mmol/g), NOVA PEG Rink Amide resin (0.67mmol/g, Merck Chemicals Ltd, Nottingham), Rink polyoxyethylenediamine-modified resin (MW 600) (0.61mmol/g) and Rink polyoxyethylenediamine-modified resin (MW 2000) (0.41mmol/g) prepared as described in Example 3.

The peptides were cleaved from the support using cocktail of TFA/TIPS/Water 95:2.5:2.5 v/v/v (5mL) except for the β Amyloid peptide where TFA/EDT/water/TIPS 94:2.5:2.5:1 v/v/v/v (5mL) for 2h followed by washing of the resin with neat TFA (2 x 3mL). Solvent was removed from the cleavage solution by rotary evaporation and the resultant crude peptide residue triturated with cold diethyl ether before lyophilizing from 10% acetic acid in water.

HPLC analysis was performed using a Varian Polaris 5µm C-18-A 150 x 4.6mm column with following conditions: Eluent A: 0.1% TFA in 10/90 ACN/water (v/v), Eluent B: 0.1% TFA in 30/70 ACN/water (v/v), using a gradient: 0-100% B in 30 min, flow rate: 1 ml/min, UV detector: 220nm, injection volume: 20µL. The results are shown in Table 1 and FIGS. 2-4.

**Table. 1. Comparative synthesis of peptides**

| **Peptide Amide** | **Support** | **Peptide Yield** | **HPLC Purity** |
|---|---|---|---|
| **VAQAAIDYING** **(Acyl Carrier Protein (65-74))** | Rink Tentagel | 196mg (83%) | **81%** |
| | Rink polyoxyethylenediamine-modified resin (MW 600) | 91.8mg (100%) | **87%** |
| | Rink polyoxyethylenediamine-modified resin (MW 2000) | 102.1mg (95%) | **87%** |
| **LMVGGVVIA (β-Amyloid (34-42))** | Rink Tentagel | 122.9mg (130%) | **39%** |
| | Rink polyoxyethylenediamine-modifiedresin (MW 600) | 67.8mg (89%) | **74%** |
| | Rink polyoxyethylenediamine-modified resin (MW 2000) | 89.3mg (98%) | **61%** |
| **AAAAAAAAAAV (Deca(alanyl)valinamide)** | Rink Tentagel | 81.6mg (86%) | **44%** |
| | NovaPEG | 48.3mg. (51%) | **36%** |
| | Rink polyoxyethylenediamine-modified resin (MW 600) | 49.4mg (67%) | **46%** |
| | Rink polyoxyethylenediamine-modifiedresin (MW 2000) | 65.7mg (75%) | **50%** |

As shown in FIG. 2 and summarized in Table 1, ACP synthesized using the different resins tested shows similar chromatographic profiles. However, ACP yields using the polyoxyethylenediamine-modified resins were higher, and the purity by HPLC was higher. In addition, as shown in FIG. 3 and summarized in Table 1, β-Amyloid Peptide Amide Fragment (34-42) synthesized using the different resins tested shows similar chromatographic profiles. However, β-Amyloid yields using the polyoxyethylenediamine-modified resins were much higher, and the purity by HPLC was higher, as significant contaminating side products are visible on the chromatogram of synthesized using Tentagel. Further, as shown in FIG. 4 and summarized in Table 1, Deca(alanyl)valinamide synthesized using the different resins tested shows similar chromatographic profiles. However, Deca(alanyl)valinamide yields using the polyoxyethylenediamine-modified resins were greater, and the purity by HPLC was higher, as significant contaminating side products are visible on the chromatogram.

### Example 8

### Comparative Synthesis of Peptide Acids Using HMP polyoxyethylenediamine-modified

### and PHB Tentagel Resins

Peptide acids to be synthesized were as follows:
a. VAQAAIDYING (Acyl Carrier Protein (ACP) (amino acids 65-74))
b. LMVGGVVIA (B Amyloid fragment (amino acids 34-42))
   Peptide synthesis and subsequent analysis was performed as described in Example 7, starting from the appropriate Fmoc-amino acid substituted HMP polyoxyethylenediamine-modified resin or PHB Tentagel resin. The results are shown in Table 2, and FIGS. 5 and 6.

**Table. 2. Comparative Peptide Acid Syntheses Using HMP Resins**

| **Peptide Acid** | **Support** | **Peptide Yield** | **HPLC Purity** |
|---|---|---|---|
| **VAQAAIDYING (ACP (65-74))** | Fmoc-Gly-PHB-Tentagel | 87.7mg (75%) | **84%** |
| | Fmoc-Gly-HMP polyoxyethylenediamine-modified resin (MW 600) | 117.3mg (100%) | **90%** |
| | Fmoc-Gly-HMP polyoxyethylenediamine-modified resin (MW 2000) | 53mg (50%) | **90%** |
| **AAAAAAAAAAV (Deca(alanyl)valine)** | Fmoc-Val-PHB Tentagel | 79.5mg (84%) | **21%** |
| | Fmoc-Val-HMP polyoxyethylenediamine-modified resin (MW 600) | 59.8mg (64%) | **35%** |
| | Fmoc-Gly-HMP polyoxyethylenediamine-modified resin (MW 2000) | 65mg (69%) | **57%** |

As shown in FIG.5 and summarized in Table 2, ACP synthesized using the different resins tested shows similar chromatographic profiles (the different sample runs are shown offset for better visualization of detail). However, ACP yields using the polyoxyethylenediamine-modified resin (MW 600) was higher, and the purity by HPLC was higher for both resins compared with Tentagel. In addition, as shown in FIG. 6 and summarized in Table 2, Deca(alanyl)valine synthesized using the different resins tested shows similar chromatographic profiles. However, the purity by HPLC was much higher, as significant contaminating side products are visible on the chromatogram, particularly for peptide synthesized using Tentagel.

### Example 9

### Preparation of Acyl Carrier Peptide Amide Using Sieber Amide Polyoxyethylenediamine-Modified Resin (MW 2000)

Preparation of the ACP peptide amide (amino acids 65-74) was carried out using the Sieber amide polyoxyethylenediamine-modified resin (MW 2000), 0.29mmol/g by Fmoc loading test on the ABI 431A peptide synthesizer, as described in Example 7. Results are given in Table 3 below and FIG. 7.

**Table 3. Peptide synthesis using Sieber Amide Polyoxyethylenediamine-Modified Resin (MW 2000)**

| **Peptide Amide** | **Support** | **Peptide Yield** | **HPLC Purity** |
|---|---|---|---|
| **VAQAAIDYING (ACP (65-74))** | Sieber Amide polyoxyethylenediamine-modified resin (MW 2000) | 110.5mg (94%) | **93%** |

As shown in FIG. 7 and summarized in Table 3, ACP synthesized using Sieber Amide polyoxyethylenediamine-modified resin (MW 2000) shows a very clean chromatographic profile, with little evidence of contaminating deletion sequences, and very good yield of 94%.

### Example 10

### Preparation of Acyl Carrier Peptide Acid using Fmoc-Gly-Trityl Polyoxyethylenediamine-Modified Resins

Preparation of peptide acid was carried out using Fmoc-Glycine derivatized polyoxyethylenediamine-modified resin (MW 600), 0.52mmol/g and polyoxyethylenediamine-modified resin (MW 2000), 0.29mmol/g on the ABI 431A peptide synthesizer as described in Example 7. Results are given in Table 4 and FIG. 8.

**Table 4. Peptide synthesis using Trityl Polyoxyethylenediamine-Modified Resin (MW 2000)**

| **Peptide Acid** | **Support** | **Peptide Yield** | **HPLC Purity** |
|---|---|---|---|
| **VAQAAIDYING (ACP (65-74))** | Trityl polyoxyethylenediamine-modified resin (MW 600) | 117.6mg (100%) | 90% |
| | Trityl polyoxyethylenediamine-modified resin (MW 2000) | 117.4mg (100%) | 93% |

As shown in FIG. 8 and summarized in Table 4, ACP synthesized using the two Fmoc-Gly-Trityl polyoxyethylenediamine-modified resins (MW 600 and MW 2000) shows similar chromatographic profiles (the different sample runs are shown offset for better visualization of detail). The chromatographic profiles are very clean, with little evidence of contaminating deletion sequences, and yields were very good at 90% and 93%, respectively.

## Claims

1. An amphiphilic solid support for solid phase synthesis, comprising a solid support and a compound according to Formula I covalently bound thereto: 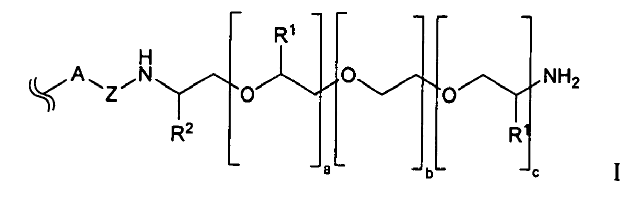 wherein A is C₅₋₁₀ aryl or heteroaryl, Z is either lower alkyl or absent, R¹ is lower alkyl, R² is H or lower alkyl, a is an integer from about 0 to about 20, b is an integer from about 5 to about 150, c is an integer from about 0 to about 20.

2. The amphiphilic solid support of claim 1, according to Formula II: 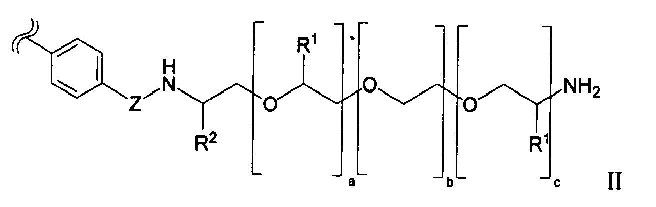 wherein Z is either lower alkyl or absent, R¹ is lower alkyl, R² is H or lower alkyl, a is an integer from 0 to about 20, b is an integer between 5 and 150, c is an integer from 0 to about 20.

3. The amphiphilic solid support of any one of claims 1-2, wherein a is from 0 to about 10, b is an integer from about 5 to about 100, c is from 0 to about 10, R¹ is methyl or ethyl and R² is methyl or ethyl.

4. The amphiphilic solid support of claim 3, wherein a is an integer from 0 to about 5, b is an integer between 5 and 60, c is an integer from 0 to about 5.

5. The amphiphilic solid support ofany one of claims 1-4, wherein the solid support comprises a polymeric matrix selected from crosslinked chloromethyl functionalized polystyrene/divinylbenzene, comprising 0.1% to 10% by weight divinylbenzene relative to the total weight of the polymer.

6. The amphiphilic solid support of any one of claims 1-5, wherein the amine loading is from about 0.01 to about 2 mmol/g dry weight of amphiphilic solid support.

7. The amphiphilic solid support of any one of claims 1-6, wherein the solid support is in the form of beads, particles, membranes, or woven or nonwoven fibers.

8. A method of preparing an amphiphilic solid support for solid phase synthesis comprising:
providing a functionalized solid support,
covalently bonding thereto a compound according to Formula III: 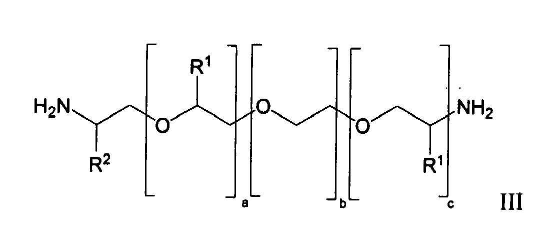
wherein R¹ is lower alkyl, R² is H or lower alkyl, a is an integer from 0 to about 20, b is an integer from about 5 to about 150, c is an integer from about 0 to about 20; wherein said covalently bonding forms a secondary amine linking the solid support and the compound of Formula III.

9. The method of claim 8, wherein a is from about 0 to about 10, b is from about 5 to about 100, c is from 0 to about 10, and R¹ and R² is methyl or ethyl.

10. The method of any one of claims 8-9, wherein the functionalized solid support comprises a material selected from crosslinked chloromethyl functionalized polystyrene, crosslinked poly(meth)acrylates, or crosslinked poly(meth)acrylamides.

11. The method of any one of claims 8-10, wherein the functionalized solid support comprises a polymeric matrix selected from crosslinked chloromethyl functionalized polystyrene/divinylbenzene, comprising 0.1% to 10% by weight divinylbenzene relative to the total weight of the polymer.

12. The method of any one of claims 8-11, wherein the solid support is in the form of beads, particles, membranes, or woven or nonwoven fibers.

13. The method of claims 8-12, wherein the solid support is in the form of particles with a mean diameter in the range between 0.1µm and 1000µm.

14. An amphiphilic solid support prepared by the method of any one of claims 8-13.

15. A method for performing solid phase synthesis, comprising:
a) providing the amphiphilic solid support according to claim 1,
b) covalently coupling to at least one amine of the compound of Formula I a linker that enables attachment of a first element or monomeric unit (e.g., an amino acid, nucleotide or sugar) in a molecule to be synthesized, wherein said linker is suitable for performing solid phase synthesis of a small molecule, oligomeric or polymeric compound, or chemical library of compounds,
c) covalently coupling to the linker an element or monomeric unit of the small molecule, oligomer or polymer, or library of compounds to be synthesized,
d) repeating the coupling steps with additional elements or monomeric units until the desired molecules have been synthesized, and
e) optionally cleaving the synthesized molecule from the amphiphilic solid support.

## Amended claims

### Amended claims in accordance with Rule 137(2) EPC.

**1.** An amphiphilic solid support for solid phase synthesis, comprising a solid support and a compound according to Formula I covalently bound thereto: 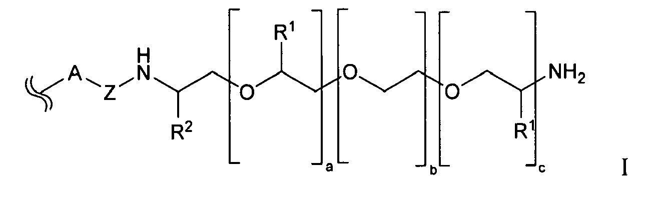 wherein A is C₅₋₁₀ aryl or heteroaryl, Z is either branched or straight chain C₁₋₁₀ alkyl moiety or absent, R¹ is branched or straight chain C₁₋₁₀ alkyl moiety, R² is H or branched or straight chain C₁₋₁₀ alkyl moiety, a is an integer from 0 to 20, b is an integer from 10 to 15 or from 35 to 45, c is an integer from 0 to 20, and the sum of a + c is an integer between 4 and 8.

**2.** The amphiphilic solid support of claim 1, according to Formula II: 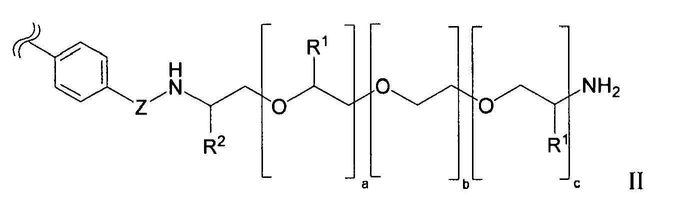 wherein Z is either branched or straight chain C₁₋₁₀ alkyl moiety or absent, R¹ is branched or straight chain C₁₋₁₀ alkyl moiety, R² is H or branched or straight chain C₁₋₁₀ alkyl moiety, a is an integer from 0 to 20, b is an integer from 10 to 15 or from 35 to 45, c is an integer from 0 to 20, and the sum of a + c is an integer between 4 and 8.

**3.** The amphiphilic solid support of any one of claims 1-2, wherein a is from 0 to 10, c is from 0 to 10, R¹ is methyl or ethyl and R² is methyl or ethyl.

**4.** The amphiphilic solid support of claim 3, wherein a is an integer from 0 to 5, c is an integer from 0 to 5.

**5.** The amphiphilic solid support of any one of claims 1-4, wherein the solid support comprises a polymeric matrix selected from crosslinked chloromethyl functionalized polystyrene/divinylbenzene, comprising 0.1% to 10% by weight divinylbenzene relative to the total weight of the polymer.

**6.** The amphiphilic solid support of any one of claims 1-5, wherein the amine loading is from 0.01 to 2 mmol/g dry weight of amphiphilic solid support.

**7.** The amphiphilic solid support of any one of claims 1-6, wherein the solid support is in the form of beads, particles, membranes, or woven or nonwoven fibers.

**8.** A method of preparing an amphiphilic solid support for solid phase synthesis comprising:
providing a functionalized solid support,
covalently bonding thereto a compound according to Formula III: 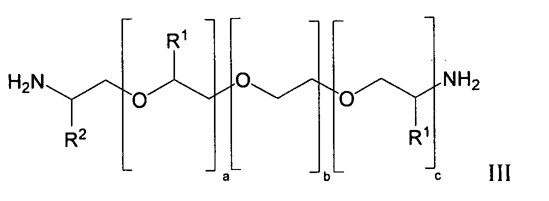
wherein R¹ is branched or straight chain C₁₋₁₀ alkyl moiety, R² is H or branched or straight chain C₁₋₁₀ alkyl moiety, a is an integer from 0 to 20, b is an integer from 10 to 15 or from 35 to 45, c is an integer from 0 to 20;
wherein said covalently bonding forms a secondary amine linking the solid support and the compound of Formula III.

**9.** The method of claim 8, wherein a is from 0 to 10, c is from 0 to 10, and R¹ and R² is methyl or ethyl.

**10.** The method of any one of claims 8-9, wherein the functionalized solid support comprises a material selected from crosslinked chloromethyl functionalized polystyrene, crosslinked poly(meth)acrylates, or crosslinked poly(meth)acrylamides.

**11.** The method of any one of claims 8-10, wherein the functionalized solid support comprises a polymeric matrix selected from crosslinked chloromethyl functionalized polystyrene/divinylbenzene, comprising 0.1% to 10% by weight divinylbenzene relative to the total weight of the polymer.

**12.** The method of any one of claims 8-11, wherein the solid support is in the form of beads, particles, membranes, or woven or nonwoven fibers.

**13.** The method of any one of claims 8-12, wherein the solid support is in the form of particles with a mean diameter in the range between 0.1 µm and 1000µm.

**14.** The use of the amphiphilic solid support according to claim 1 for solid phase synthesis.
